# EUROPEAN PATENT SPECIFICATION

(11) **EP 2 781 199 B1**
(45) Date of publication and mention of the grant of the patent: **28.12.2016**
(21) Application number: 13187799.5
(22) Date of filing: 08.10.2013
(51) Int. Cl.: A61B 34/20

(54) **Apparatus for the orientation and positioning of surgical instruments and of implantation prostheses in a bone seat**
Vorrichtung zur Ausrichtung und Positionierung von chirurgischen Instrumenten und Implantationsprothesen in einen Knochensitz
Appareil pour l'orientation et le positionnement d'instruments chirurgicaux et l'implantation de prothèses dans un siège d'os

(30) Priority: 21.03.2013 IT MI20130432
(43) Date of publication of application: 24.09.2014
(73) Proprietor: Dial Medicali S.r.l., 20129 Milano (IT)
(72) Inventor: Lo Iacono, Giorgio, 20126 Milano (IT); Lo Iacono, Diego, 20162 Milano (IT); Fantauzzo, Domenico, 20126 Milano (IT)
(74) Representative: Faggioni, Marco

(56) References cited:
- WO-A1-99/62401
- US-A1- 2005 182 320
- US-A1- 2006 094 958
- US-A1- 2011 313 288

## Description

### FIELD OF THE INVENTION

The present invention relates to an apparatus which may be used in surgical operations in which artificial prosthesis replacing bone joints are applied. More precisely, it is an apparatus apt to allow the correct and quick angular orientation of surgical instruments and of implantation prostheses of the acetabulum component in a respective bone seat, in prosthetic surgery of the human hip.

The aim of the apparatus according to the invention is to offer a means for quickly and precisely guiding the surgeon during the pelvis prosthesis operation, providing in real time the indications for reaching a target angular orientation, previously pre-set in a pre-surgery step.

### BACKGROUND PRIOR ART

Surgical operations wherein bone joints are replaced by artificial prostheses, as in particular the human hip joint, are known from a long time and widely used.

As known, while on the side of artificial prosthesis manufacturing many studies and great progress have been made, on the side of the application of these prostheses, medical expertise and the surgeon's manual dexterity remain paramount. However, that means that application defects may arise - as they have in fact really arisen, even though sporadically and due to inadequate operating conditions - which have caused a painful condition to the patient, walking difficulties or even the need to repeat the surgical operation to change the implant positioning.

Scientific research has highlighted that the main source of mechanical complications is especially an incorrect positioning of the prostheses. As a result, attempts have been made to improve surgeons' performances through more positioning information.

This problem has become more evident in more recent years, in which techniques and implantation methods have been studied which resort to numerically-controlled biotechnology. In jargon, robot-controlled navigation is meant. An in-depth explanation of these concepts and of the systems currently on the market is reported in *"*Robotic assisted total hip arthroplasty using the MAKO platform - Authors: Rupesh Tarwala & Lawrence D. Dorr - Online publication: 5 July 2011 by Springer Science+Business Media, LLC 2011*"*.

Among the most recent studies, which have led to the accomplishment of apparatuses suited to the purpose, US patent application no. 2012/0022406 is cited, which represents the closest prior art to the invention, in which a method and a system for determining the orientation of a surgical instrument with respect to a patient's bone are disclosed, such as in particular the pelvis, due to the use of two position sensors, a first one fastened in a patient's pre-set bone position and a second one made integral with a surgical instrument.

The first sensor must be fastened in a clearly pre-set position and with a clearly pre-set orientation with respect to a reference plane, representative of the space orientation of the bone, which plane is defined identifying beforehand at least three reference positions in the bone. For this purpose the system also comprises a mechanical device which is physically anchored to the pelvis in the above-said reference positions and which locates - with an arm thereof - the position and the fastening orientation of the first sensor.

The first and the second sensor are apt to continuously detect their own position and to transmit this information to a calculator which, based on the initial data on the positioning of the first sensor with respect to the patient's bone, provides in a display the information necessary for the surgeon to be able to instantly check the exact angular orientation of the surgical instrument with respect to the patient's bone. Such an apparatus, despite allowing to provide the surgeon with objective and real information of the exact orientation of the surgical instrument with respect to the patient's bone, still suffers from some remarkable drawbacks, and in particular:
- the need to locate three reference positions on the patient's bone, and to fasten on the patient's body the device which allows to determine the position of the first sensor significantly complicates the use of the system and causes a remarkable lengthening of the operation times, with respect to traditional surgery, with a consequent greater patient burden;
- the mechanical device for determining the position of the first sensor must furthermore be adapted, each time, to the patient's specific dimensional ratios, preferably in a pre-surgery step and this, in specialised surgery rooms which perform multiple similar operations in sequence, requires the availability of a certain number of devices as well as the organisation of a correct flow thereof between the moment in which they are calibrated for a single patient and the one in which they are actually used on such a patient in the surgery room, with evident problems of defining correct logistics and sterilisation;
- the need to implant the first sensor on the patient's bone in a univocally, *a priori* determined position, limits the surgeon's freedom of action with respect to the placement of said sensor in other bone sites better suited from the patient's point of view or more comfortable in the light of the adopted surgery technique. It is hence necessary to have a supply of different mechanical devices for determining the position of the first sensor, to be selected depending on the specific surgery technique adopted;
- the fact that the information data on the orientation of the surgical instrument are supplied on an outer display makes a continuous gaze shift of the surgeon from the operating field to the display necessary, introducing an element of disturbance and of fatigue for the surgeon.

US-2005/182320 and US-2006/094958 are other previous documents of the prior art which disclose apparatuses and arrangements for ascertaining and calibrating angular orientation of surgical instruments and of implantation prostheses in a respective bone seat.

### PROBLEM AND SOLUTION

The problem at the basis of the invention is hence that of overcoming the above described drawbacks, suggesting an apparatus for the positioning of the surgical instruments and prostheses which has a limited cost and allows to effectively perform in a short time the preliminary calibration.

The invention is defined in claim 1. Preferred embodiments are recited in the dependent claims.

### BRIEF DESCRIPTION OF THE DRAWINGS

Further features and advantages of the invention are in any case more evident from the following detailed description of a preferred embodiment of the same, given purely as a non-limiting example, and illustrated in the attached drawings, wherein:
fig. 1 is a schematic front view of the bone part of a human pelvis, which shows also a prosthesis of an acetabulum cup during its installation for replacing the normal acetabulum housing the femur head (not shown);
fig. 2 is a front perspective view of the bone part of a human pelvis, wherein also the ideal reference planes are shown, for positioning the prosthesis;
fig. 3 is a view similar to that of fig. 1, wherein the direction of insertion of the prosthesis is shown;
fig. 4 is a lateral perspective view of the bone part of a human pelvis, wherein both the ideal reference plane and the direction of insertion of the prosthesis are shown;
fig. 5 is a lateral schematic view of the pelvis bone, when the patient is positioned on an operating plane;
fig. 6 is a schematic view fully similar to that of fig. 5, but which furthermore shows the effects of the change of the crosswise angle of the tilting plane on the operating angles, with the patient positioned on the operating plane;
fig. 7 shows two diagrams which illustrate, upon the changing of the γ angle of the tilting plane, the change of the anteversion angle α and of inclination angle β, respectively;
figs. 8A and 8B schematically show how the operating angles may vary during the operation following pelvis movements;
fig. 9 shows in a perspective view the central control unit of the apparatus of the present invention;
fig. 10A shows the position sensor of a first embodiment of the apparatus of the present invention, to be fastened on the patient;
fig. 10B shows the position sensor of a second embodiment of the apparatus of the present invention, to be fastened on the patient;
fig. 11 shows the support clamp of a surgical instrument of the apparatus of the present invention;
fig. 12 shows the use of clamp of fig. 11 in association with the central unit of fig. 9;
fig. 13 shows compasses for the detection of the crosswise angle of the tilting plane of the apparatus of the present invention, in association with the central unit of fig. 9; and
fig. 14 shows the rear side of the central control unit of the apparatus of the present invention.

### DETAILED DESCRIPTION OF THE PREFERRED EMBODIMENT

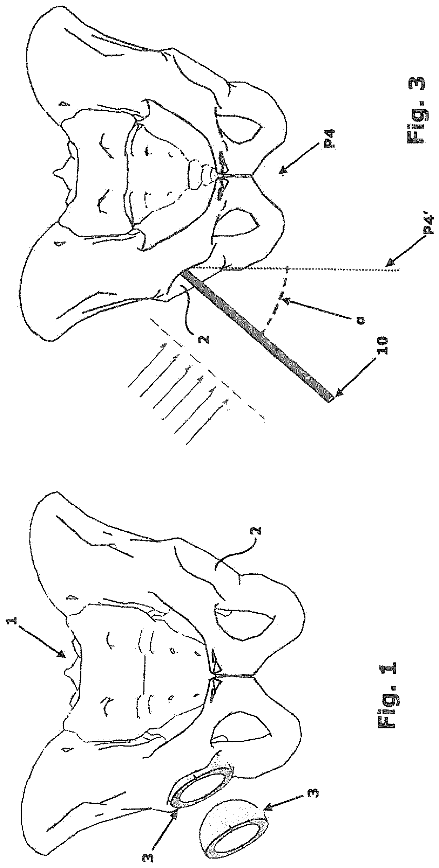

Fig. 1 shows pelvis bone 1, wherein the two acetabula 2 are schematically shown; in correspondence of the acetabulum which is shown on the left hand side of the drawing, an artificial prosthesis 3 of the acetabulum cup must be inserted.

As already mentioned, it is well known *(see for example "*The accuracy of free-hand cup positioning" - Authors: G. Saxler, A. Marx, D. Vandevelde and others - Online publication: 15 May 2004, by Springer-Verlag*)* that the incorrect positioning of said acetabulum cup may imply a series of complications such as to impair the entire implant. Therefore, the apparatus according to the invention is meant to support the surgeon in the operation of correctly introducing and fastening this prosthesis 3 on pelvis 1, during a total or partial hip prosthesis surgery.

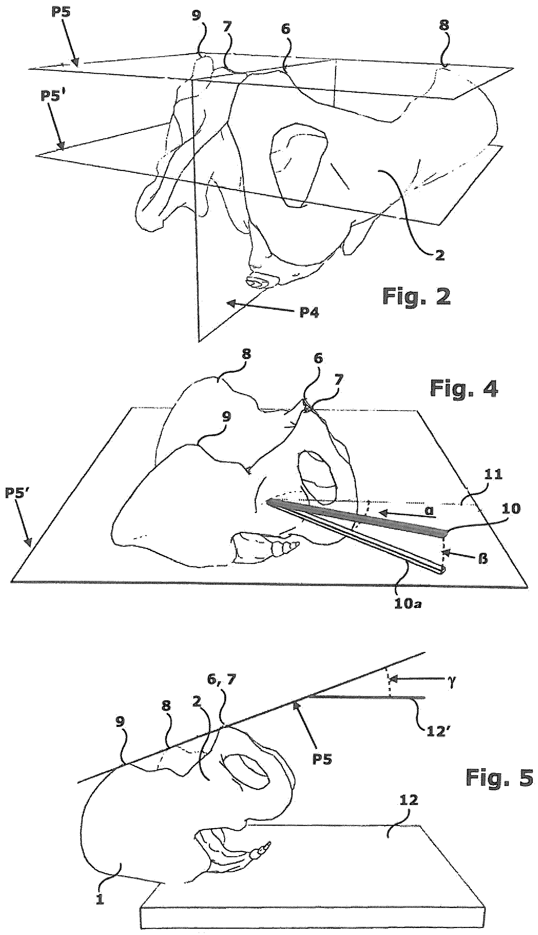

Making now reference to fig. 2, the correct direction of insertion of the prosthesis, i.e. the direction according to which both the surgical instrument which prepares the seat in the bone and the surgical instrument which then inserts the prosthesis into such seat must be arranged, may be obtained by the identification of two angles calculated with respect to two anatomical pelvis reference planes: the "pelvis plane of symmetry", referred to as P4, and the "zero-tilt plane" also called "tilting plane", referred to as P5. Fig. 2 highlights how these two planes P4 and P5 are mutually orthogonal. "Tilting plane" P5 is the imaginary plane containing the two upper points 6 and 7 of the pubic symphysis and the two iliac crests 8 and 9.

For greater ease of understanding, such tilting plane may be translated downwards (position P5'), until passing through a central point of the two acetabula 2, at least one of which makes up the insertion site of acetabulum cup 3.

Figures 3 and 4 show the ideal direction 10 which actually represents the target for the correct positioning of acetabulum cup 3.

This ideal anatomical direction for the insertion of the acetabulum cup may be defined in a univocal manner through the identification of the anatomical inclination angle α and of the anatomical anteversion angle β. The anatomical anteversion angle β is the angle formed between the ideal direction 10 and the projection 10a thereof on tilting plane P5', while the anatomical inclination angle α is the angle lying on tilting plane P5', formed between the projection 10a of the ideal direction 10 and the line 11 of intersection between symmetry plane P4 and tilting plane P5'.

Figure 4 highlights how the anteversion angle β may be measured simply through a pelvis section made according to a plane which is perpendicular to tilting plane P5' - such as the symmetry plane P4 (trace P4' in fig. 3) - and is inclined with respect to this symmetry plane P4 by an angle equal to the anatomical inclination angle α. When the patient is positioned supine on a planar surface 12 (for example on the bed for radiographs or the operating bed, as schematically shown in Fig. 5) the pelvis takes up a position in which tilting plane P5 cuts across plane 12 (or a reference plane 12' parallel thereto) according to an angle γ generally called "tilting angle".

During the surgery it is not possible to detect exactly tilting plane P5 or P5' and it is hence difficult for the surgeon to use the anatomic inclination angle and the anatomic anteversion angle as references for the correct positioning of acetabulum cup 3. It is hence necessary to transform the two above-cited anatomic angles α and β into two different angles α' and β' (called in the following "surgery angles") which use the operating bed as reference system.

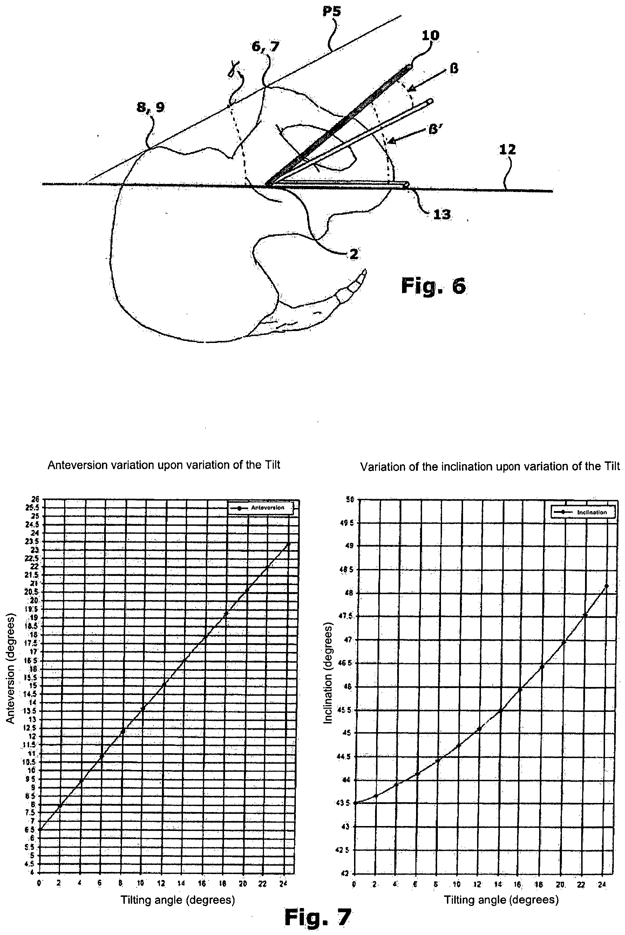

Fig. 6 highlights how the transformation of the two original anatomic angles into the different angles α' and β' is definitely affected by tilting angle γ.

The diagrams of fig. 7 highlight how a pair of surgery angles (for example: anteversion angle = 6.5° and inclination angle = 43.5°) originate different surgery inclination and anteversion angles depending on the value of tilting angle γ.

In the light of this premise, it must be reminded again that tilting angle γ depends on anatomical factors (pelvis morphology, patient's physique), on the position taken up by the patient on the bed, in turn depending also on the type of contact surface. Some patient positioning techniques on the bed, which allow to maintain a constant tilting angle in subsequent patient positioning instances are described for example in *"*A Validation Model for Measurement of Acetabular Component Position, by Aamer Malik, MD,* Zhinian Wan, MD,* Branislav Jaramaz and others, in The Journal of Arthroplasty Vol. 25 No. 5 2010*" .* Such techniques may prove extremely useful, since they allow to estimate through a preparatory X-ray - as everybody knows, an extremely quick and inexpensive operation - the initial patient tilting angle with respect to the surgery table.

Estimating the tilting angle through a simple X-ray *(as described in* Risk Factors for Cup Malpositioning, by Mark C. Callanan MA, Bryan Jarrett BS, Charles R. Bragdon PhD and others, published online: 18 August 2010 by The Association of Bone and Joint Surgeons*)* and knowing or estimating the anatomical target angles, it is hence possible to calculate the "surgery target angles", i.e. the inclination and anteversion angles with respect to reference plane 12 to be used for the positioning of the acetabulum cup.

The calculation of such surgery angles must of course take into account also the patient's initial position with respect to reference plane P5, since the patient's pelvis could not be positioned correctly with respect to reference plane 12.

Moreover, the surgery angles may vary during surgery following pelvis movements, with respect to the initial position, due to various types of ways of access for the implantation of a hip prosthesis or due to the patient's voluntary or involuntary movements.

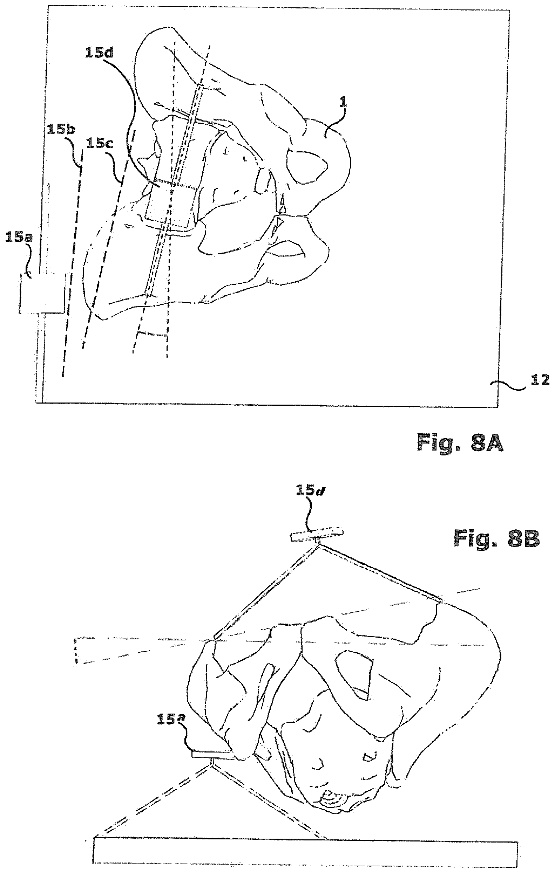

Figs. 8A and 8B schematically show this possibility: it can be noticed how, resting on pelvis 1 a position detection instrument 15 (such as the one subject of the present invention, better described in the following), it moves from a zero position, referred to as 15a, to subsequent different positions 15b, 15c, 15d, following the pelvis movements with respect to reference plane 12, and this both in the horizontal plane (fig. 8A) and in the vertical plane (fig. 8B).

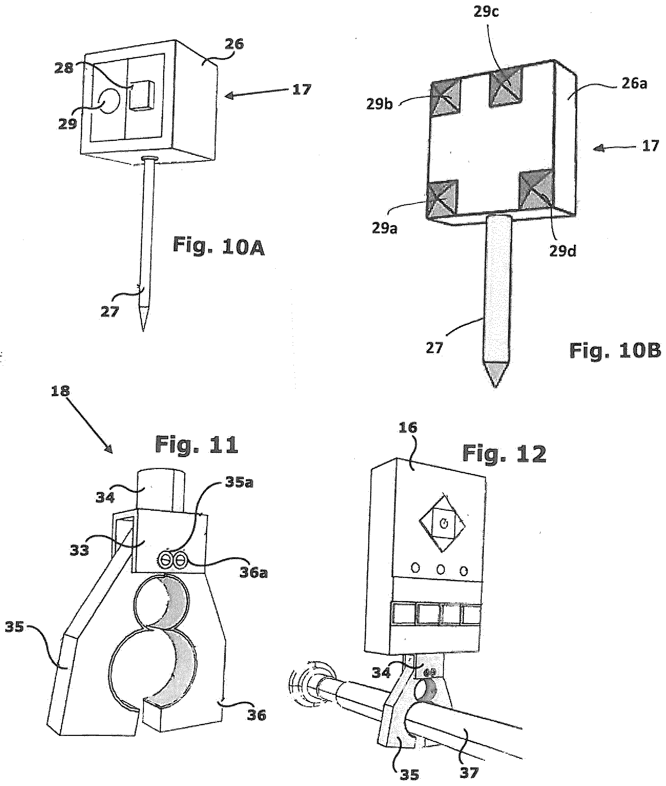

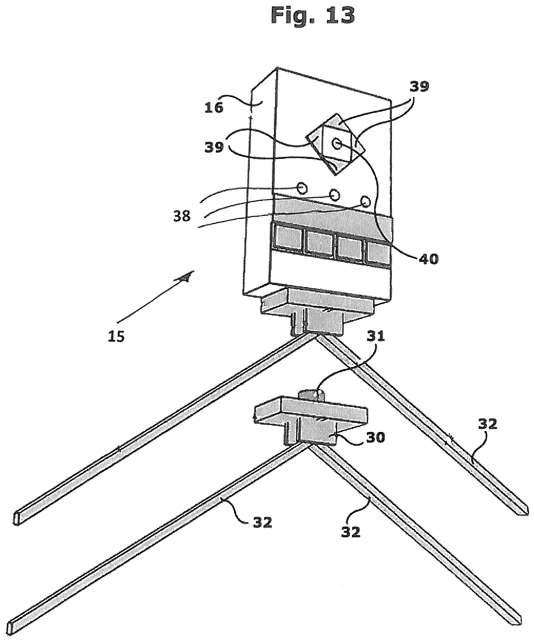

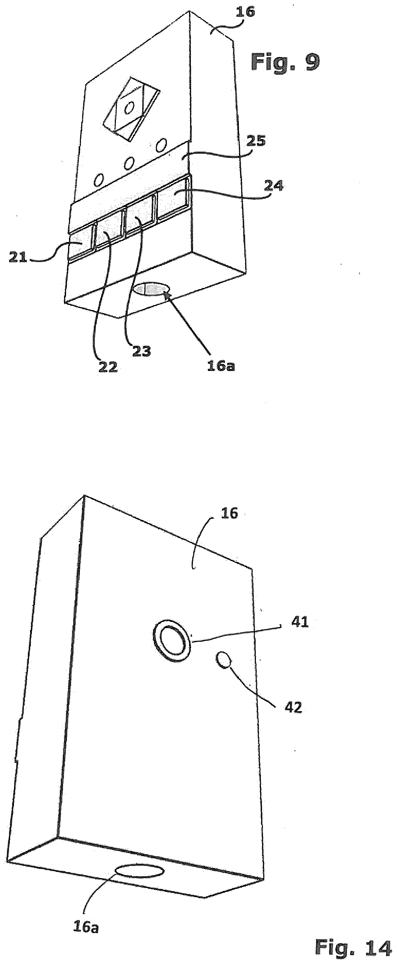

In order to achieve the desired object, and as illustrated in figs. 9 to 13, the invention provides an apparatus which comprises a central unit 16 (fig. 9), which acts as moving sensor, which makes up the main control device, a fixed sensor 17 for the detection of the intra-surgery movements provided with a surgery pin 27 (fig. 10) for the fastening on the patient's bone affected by the surgery, a compasses-like instrument 15 (fig. 13) for the detection of the patient's initial position, and a clamp 18 (fig. 11) for guiding a surgical implant instrument.

More precisely, fixed sensor 17 consists of a sensor box 26 and of a surgery pin 27. With a first end thereof, pin 27 is associated - through a well-known surgical technique - with the patient's pelvis, so that it integrally accompanies every movement thereof, both in translation and in rotation, accurately and precisely. At the other end of pin 27 box 26 is then fastened which, of course, in turn follows every movement of the patient's pelvis.

In a first embodiment of the invention, illustrated in fig. 10A, box 26 embeds position detection means and signalling means of the detected position. On the front of the box an activation button 28 and a signalling light 29 are available.

The position detection means and the detected position signalling means within box 26 belong to the prior art, according to which different possible technical ways are provided for providing an accurate position detection, i.e. a detection which univocally determines the position and the orientation in space of the fixed sensor 17 with the desired precision degree, for example using gyroscopes, accelerometers or magnetometers. Such technical ways are not disclosed here in detail since they do not fall within the scope of protection of the present invention.

In a second embodiment of the invention, illustrated in fig. 10B, box 26 contains no intelligent function, but represents a simple support 26a of 4 or more blocks of reflective material 29a, 29b, 29c, 29d, so as to allow the remote detection of the exact position of said support 26a, and hence of the pelvis during surgery, through the analysis of a light radiation reflected by said blocks 29, as better described in the following.

The compasses-like instrument 15 is in turn in the shape of a support 30 to which two legs 32 are connected below, which legs may be spread apart and adjusted in position in the way better described in the following. Above, support 30 is provided with a tang 31, meant to be inserted precisely (and in a single position) into the hole 16a formed in the lower face of central unit 16, in the way highlighted in fig. 13.

Clamp 18 consists of a base 33, from which a tang 34 projects upwards 34 (fully identical to tang 31) for the coupling with central unit 16. To the lower part of base 33 two clamp jaws 35, 36 are jointed; at least one of the jaws, but preferably both, are pivoted at their top on respective pins 35a and 36a.

Between these jaws it is meant to be housed the stem 37 of the surgical instruments used - in a way fully known per se - firstly to form in the pelvis bone the seat for the artificial prosthesis 3 of the acetabulum cup, and successively to perform the fastening of such prosthesis in said seat.

Finally, the central control unit/moving sensor 16 - which is meant to be alternatively coupled with the support 30 of the compasses or with the base 33 of clamp 18 - is provided with a position detection system fully similar to the one described in connection with the first embodiment of fixed sensor 17 and furthermore with a memory and processing unit (the technical details of which are not part of the present invention, since they are fully known per se to an electronic technician) which is capable of storing predefined information on the surgery angles and on the angle of the tilting plane and hence of collecting the position information coming from the detection and position means housed in the same central unit 16 and in the box 26 of fixed sensor 17, through said detected position signalling means , in order to supply the surgeon with indications on the correct orientation of the surgical instruments.

In the second embodiment of the present invention, and as schematically illustrated in fig. 14, central unit 16 comprises also a source of light radiation, for example a LED 42, and a camera 41 apt to capture the image of blocks 29a, 29b, 29c, 29d which reflect the light produced by LED 42. The analysis of such image, through a suitable processing programme integrated in central unit 16, allows to detect precisely the position of support 26a and hence that of fixed sensor 17.

The operation of the apparatus illustrated above is now described together with the implantation method of a prosthesis, which method may be divided into 5 step for ease of exposition.

### 1st step - definition of the target

This first step is purely a planning one: it may be performed before the surgical operation and provides the definition of surgery angles α' and β' which will be the target to be reached during the surgical operation. As already mentioned in the introduction, such planning must take into account various pieces of information and precisely both of the reference anatomical angles α and β and of the compensation determined by an initial tilt angle γ other than 0.

Firstly it must be said that this step may differ depending on the different possible surgery approaches; however, it is understood that the invention can be applied to all the approaches available today.

The anatomical inclination angle α, the anatomical anteversion angle β and the initial tilt angle γ are calculated through the physician's direct intervention, in a pre-surgery step, taking into account anatomical parameters deriving from general anatomical studies as well as from direct detections on the patient, for example detecting the tilt angle by X-rays, and assuming an ideal positioning of the patient (as shown in Fig. 5), that is, supine, and with nil lateral rotation with respect to the surgery plane.

The surgery angles thus calculated are then directly introduced in the central control unit 16, schematically shown in Fig. 9, using for this purpose buttons 21, 22, 23 and 24 and display 25, arranged on the front side of central unit 16.

### 2nd step - calibration of the devices and positioning of the fixed sensor

The second step of use of the apparatus of the invention comprises to position the patient supine and aligned with respect to the operating table and to perform an initial calibration of central unit 16 and of sensor 17 for the correction of the intra-surgery movements.

The calibration of the two devices 16 and 17 consists of the positioning of both devices in a pre-set position with respect to the operating table, called "calibration position", and of the activation of one of the buttons (21, 22, 23 or 24) of central unit 16 which enables the two devices to store such position as initial reference position.

Surgical pin 27 is hence fastened to the patient's pelvis and the box 26 of position sensor 17 is then joined thereto and made integral therewith. Thereby, every movement of the patient's pelvis - also in an intra-surgery step and regardless of the fact that it is a movement imposed by the outside or caused by the patient himself - is detected by the position detection system of sensor 17 and hence transmitted to central unit 16, in the first embodiment of the invention, or directly detected by central unit 16 through the camera 41, whenever it is in visual contact with reflecting blocks 29 of sensor 17, in the second embodiment of the invention. There is no compulsory location where to enter the pin, which is left to the surgeon's free choice depending on the adopted technique or on the patient's conditions.

There follows a communication between the two units to define a start position of the surgical operation control, or zero position. Due to this calibration procedure, central unit 16 is capable of calculating the changes of its angular position on the three axes, both with respect to the reference system consisting of the operating bed, and with respect to the position of the patient's pelvis, which position as a matter of fact is defined by the position of sensor 17. Such estimate is possible due to the presence of dedicated electronic devices for this purpose, within both devices.

### 3rd step - static correction of the patient's initial position

This third step has the purpose of compensating mistakes caused by an imprecise positioning of the patient with respect to the reference system made up of the operating bed. This compensation of the initial positioning mistakes is obtained by simply mechanically coupling the central unit 16 with the compasses-like device 15, as shows in Fig. 13, and pointing the two ends of the compasses on the two iliac crests (Figs. 8A and 8B) and then pushing that of buttons 21, 22, 23, 24 of central unit 16, provided for this purpose.

At this point the central unit detects the inclination thereof with respect to the theoretical horizontal lying plane of the patient and directly receives or detects the position of sensor 17; using such information, the central unit is capable of defining a new reference plane for the subsequent positioning operations.

### 4th step - coupling of the surgical instruments and positioning thereof according to the predefined surgery angles

In this last step of use of the apparatus, the stem 37 of the surgical instrument used on each occasion (for example a mill for the forming of the bone seat and an impactor for the insertion of the prosthesis into said seat) is introduced between jaws 35 and 36 of clamp 18 and tightened between the same without any slack. The central unit 16 is then mechanically coupled with clamp 18, introducing the tang 34 of clamp 18 into hole 16a formed in the base of the central unit 16, as shown in Fig. 12. Thereby a stable mechanical coupling between central unit 16 and the surgical instrument is accomplished extremely quickly, so that the angular orientation of said instrument is identical to that of central unit 16.

At this point the surgeon, after having positioned the surgical instrument in the desired location is able to adjust with the utmost precision and rapidity the angular position of such instrument in alignment with the predefined operating angles, simply observing the front surface of central unit 16 on which indicators of the angular position and of the target positioning are provided, which surface is in a maximum visibility position, above the surgeon's hand and in front of him/her, without the surgeon having to shift his/her gaze from the operating field.

Preferably, the above-said indicators are all of the light type and, in particular, the angular positioning ones consist of four arrow-shaped LEDs 39 arranged according to the four cardinal directions, the lighting of which indicates the direction in which the surgeon must incline the instrument to get closer to the desired angular positioning, while the indicator of the target positioning consists of a LED 40 centrally arranged between the four LEDs 39, which activates itself only when the surgical instrument is correctly aligned according to the predefined surgery angles.

LEDs 38 are instead used to supply further information to the surgeon, such as for example warning of problems linked to the incorrect visual contact between camera 41 and reflective support 26a.

In the light of what has been stated above, it should be clear that the target positioning provided by the central unit takes into account both the initial parameters provided in the above-described first step, and the initial positioning mistakes of the patient detected in the third step, and finally it compensates in real time any movements of the patient's pelvis detected by sensor 17.

The system is apt to compensate also significant pelvis rotations according to the three axes, hence guaranteeing the compatibility of the apparatus of the invention with any operating technique, including those which provide a rotation of the patient from the supine position to a lateral position.

From the preceding description it is evident how the apparatus of the present invention has fully achieved the appointed object. Through the combined use of the different interfunctional devices which make up said apparatus, as a matter of fact the surgeon can quickly establish the most correct positioning of the surgical instruments, depending on the desired parameters, even specifically for the individual patient, and regardless of the position which the patient may take up during the operation.

The sensor 17 for the detection of the patient's position may be fastened in any point of the bone affected by the implantation procedure, hence leaving full freedom of choice to the surgeon, depending on the surgical technique adopted, on the chosen position for the patient and on the conditions of the same.

Due to the arrangement of the visual positioning indicators directly on the central unit and to the fact that such unit is fastened onto the stem of the surgical instruments in such a way that the indicators face the operator, the surgeon can follow easily and immediately the visual indications which guide him/her towards the correct angular positioning of the instrument, while maintaining under control the operating area. This arrangement allows to reduce both the time necessary for the fine-positioning of the surgical instruments and the surgeon's fatigue, all to the patient's advantage.

The apparatus causes a minimum burden towards the patient and provides - unlike the above-described prior art - extremely fast initial calibration operations, considering that such operations are limited to the initial calibration operations of the central unit and to the detection of the actual inclination referred to two sole significant pelvis points (iliac crests 8 and 9), instead of the detection of the mutual distances between three patient's points, as provided by the apparatus of the above-described prior art.

Finally, the cost of the apparatus of invention is extremely low compared to the extremely high costs of the other assisted positioning techniques of the prior art.

However, it is understood that the invention must not be considered limited to the specific embodiment illustrated above, which represents only an exemplifying embodiment of the same, but that different variants are possible, all within the reach of a person skilled in the field, without departing from the scope of the invention, which is defined solely by the following claims.

## Claims

1. Apparatus for the fast angular orientation of a surgical instrument with respect to an implantation seat of a prosthesis in a patient's bone, of the type comprising a moving sensor (16), which can be associated with said surgical instrument, and a fixed sensor (17), which can be integrally fastened to said bone during a surgical operation, said moving sensor (16) and said fixed sensor (17) being provided with position detection means and with means configured to signal the detected position to a central memory and processing unit, said central memory and processing unit comprising calibration means which are configured to detect the position of said moving sensor (16) when it is arranged in a fixed position with respect to a line passing through two known points of said bone and, simultaneously, configured to detect the position of said fixed sensor (17) integrally fastened to said bone, and which are configured to record such a position as the initial work position **characterised in that** said apparatus further comprises a compasses-like device (15) provided with coupling means (30, 31) for steadily and reversibly coupling with said moving sensor (16) and with spreadable arms (32) the free ends of which are configured to be rested onto said two known points of said bone for thereby arranging the moving sensor (16) into a fixed relationship with respect to the line passing through said points.

2. Apparatus as claimed in claim 1, wherein said central memory and processing unit is associated with said moving sensor (16).

3. Apparatus as claimed in claim 2, furthermore comprising a clamp-like device (18) provided with coupling means (33, 34) for steadily and reversibly coupling with said moving sensor (16) and with jaw-like means (35, 36) for locking into position said surgical instrument.

4. Apparatus as claimed in claim 3, wherein said central memory and processing unit comprises indicators of the moving sensor (16) angular positioning and of a target angular positioning, said indicators being provided on a surface of the moving sensor (16) facing the surgeon who grips the surgical instrument, when said moving sensor (16) and said surgical instrument are steadily associated through said clamp (18).

5. Apparatus as claimed in claim 4, wherein said indicators are light type indicators, the indicators of the moving sensor (16) angular positioning consisting of four arrow-shaped LEDs (39) arranged according to the four cardinal directions, the activation of which indicates the direction(s) where the surgeon must incline the instrument to get closer to the target angular positioning, and the indicator of the target angular positioning consisting of a LED (40) which is activated when the surgical instrument is correctly aligned according to the target angular positioning.

6. Apparatus as claimed in claim 3, wherein said coupling means (30, 31; 33, 34) consist of tang/hole couplings with a univocal coupling position.

7. Apparatus as claimed in any one of the preceding claims, wherein said central memory and processing unit comprises initial calibration means which detect the position of said mobile sensor (16) and of said fixed sensor (17) when they are arranged, in a pre-set orientation, on a same reference plane outside the patient and such a position is recorded as the initial reference position thereof.

8. Apparatus as claimed in claim 2, wherein said fixed sensor (17) is integrally fastened onto said bone during a surgical operation through a surgical pin.

9. Apparatus as claimed in claim 8, wherein said fixed sensor (17) comprises a box (26) in which independent means for the detection of its own position and means for signalling such detected position to said central memory and processing unit are contained.

10. Apparatus as claimed in claim 8, wherein said fixed sensor (17) comprises a support (26a) provided with multiple reflecting blocks (29a, 29b, 29c, 29d), and said central memory and processing unit furthermore comprises a source of a light radiation (42), a camera (41) apt to capture the images of said reflecting blocks (29a, 29b, 29c, 29d) and a processing programme for analysing said images and providing a remote detection of the exact position of said fixed sensor (17).

11. Apparatus as claimed in any one of the preceding claims, wherein said bone is the pelvis bone of a human being.

12. Apparatus as claimed in claim 11, wherein said two known points of the pelvis bone are the iliac crests (8, 9).

## Patentansprüche

1. Vorrichtung für die schnelle winkelförmige Ausrichtung eines chirurgischen Instruments in Bezug auf einen Implantationssitz einer Prothese in einem Knochen eines Patienten, des Typs umfassend einen sich bewegenden Sensor (16), der mit dem chirurgischen Instrument assoziiert sein kann, und einen fixierten Sensor (17), der integral an dem Knochen während einer chirurgischen Operation befestigt sein kann, wobei der sich bewegende Sensor (16) und der fixierte Sensor (17) mit Positionsdetektionsmitteln und mit Mitteln, die konfiguriert sind, um die detektierte Position an eine zentrale Speicher- und Verarbeitungseinheit zu signalisieren, bereitgestellt sind, wobei die zentrale Speicher- und Verarbeitungseinheit Kalibrierungsmittel umfasst, die konfiguriert sind, um die Position des sich bewegenden Sensors (16) zu detektieren, wenn er in einer fixierten Position in Bezug auf eine Linie führend durch zwei bekannte Punkte des Knochens, angeordnet ist, und gleichzeitigt konfiguriert sind, um die Position des fixierten Sensors (17), integriert an dem Knochen befestigt, zu detektieren, und welche konfiguriert sind, um eine solche Position als die Arbeitsanfangsposition aufzuzeichnen, **dadurch gekennzeichnet, dass** die Vorrichtung ferner eine kompassartige Vorrichtung (15) umfasst, die mit Kopplungsmitteln (30, 31) zum steten und reversiblen Koppeln mit dem sich bewegenden Sensor (16) bereitgestellt ist, und mit spreizbaren Armen (32), deren freie Enden konfiguriert sind, um auf den zwei bekannten Punkten des Knochens zu ruhen, um dadurch den bewegenden Sensor (16) in einer fixierten Beziehung in Bezug auf die Linie führend durch die Punkte angeordnet zu sein.

2. Vorrichtung nach Anspruch 1, wobei die zentrale Speicher- und Verarbeitungseinheit mit dem sich bewegenden Sensor (16) assoziiert ist.

3. Vorrichtung nach Anspruch 2, weiter umfassend eine klammerartige Vorrichtung (18), die mit Kopplungsmitteln (33, 34) zum stetigen und reversiblen Koppeln mit dem sich bewegenden Sensor (16) und mit klemmartigen Mitteln (35, 36) zum Verriegeln des chirurgischen Instruments in Position bereitgestellt ist.

4. Vorrichtung nach Anspruch 3, wobei die zentrale Speicher- und Verarbeitungseinheit Indikatoren des winkelförmigen Positionierens des sich bewegenden Sensors (16) und einer winkelförmigen Zielpositionierung umfasst, wobei die Indikatoren auf einer Oberfläche des sich bewegenden Sensors (16), zugewandt dem Chirurgen, der das chirurgische Instrument greift, bereitgestellt sind, wenn der sich bewegende Sensor (16) und das chirurgische Instrument stetig durch die Klammer (18) assoziiert sind.

5. Vorrichtung nach Anspruch 4, wobei die Indikatoren lichtartige Indikatoren sind, wobei die Indikatoren des winkelförmigen Positionierens des sich bewegenden Sensors (16) aus vier pfeifförmigen LEDs (39) bestehen, angeordnet gemäß den vier Himmelsrichtungen, wobei die Aktivierung derselben die Richtung(en) angibt, in die der Chirurg das Instrument neigen muss, um näher an die winkelförmige Zielpositionierung zu kommen, und wobei der Indikator der winkelförmigen Zielpositionierung aus einer LED (40) besteht, die aktiviert wird, wenn das chirurgische Instrument korrekt gemäß der winkelförmigen Zielpositionierung ausgerichtet ist.

6. Vorrichtung nach Anspruch 3, wobei das Kopplungsmittel (30, 31; 33, 34) aus Dorn/Loch-Kopplungen mit einer unzweideutigen Kopplungsposition besteht.

7. Vorrichtung nach einem der vorangehenden Ansprüche, wobei die zentrale Speicher-und Verarbeitungseinheit Anfangskalibrierungsmittel umfasst, die die Position des mobilen Sensors (16) und des fixierten Sensors (17), wenn sie angeordnet sind, detektiert, in einer vorgegebenen Orientierung, auf einer gleichen Bezugsebene außerhalb des Patienten, und wobei eine solche Position als die Anfangsbezugsposition derselben aufgezeichnet wird.

8. Vorrichtung nach Anspruch 2, wobei der fixierte Sensor (17) integral an dem Knochen während einer chirurgischen Operation durch einen chirurgischen Stift befestigt ist.

9. Vorrichtung nach Anspruch 8, wobei der fixierte Sensor (17) eine Box (26) umfasst, in der unabhängige Mittel für die Detektion seiner eigenen Position und Mittel zum Signalisieren einer solchen detektierten Position an die zentrale Speicher- und Verarbeitungseinheit enthalten sind.

10. Vorrichtung nach Anspruch 8, wobei der fixierte Sensor (17) einen Träger (26a) umfasst, bereitgestellt mit mehreren reflektierenden Blöcken (29a, 29b, 29c, 29d), und wobei die zentrale Speicher- und Verarbeitungseinheit ferner eine Lichtstrahlungsquelle (42), eine Kamera (41), die geeignet ist, um die Bilder der reflektierenden Blöcke (29a, 29b, 29c, 29d) aufzunehmen, und ein Verarbeitungsprogramm zur Analyse der Bilder und zum Bereitstellen einer Ferndetektion der exakten Position des fixierten Sensors (17) umfasst.

11. Vorrichtung nach einem der vorangehenden Ansprüche, wobei der Knochen der Beckenknochen eines Menschen ist.

12. Vorrichtung nach Anspruch 11, wobei die zwei bekannten Punkte des Beckenknochens die Beckenkämme (8, 9) sind.

## Revendications

1. Appareil pour l'orientation angulaire rapide d'un instrument chirurgical par rapport à un siège d'implantation d'une prothèse dans un os du patient, du type comprenant un capteur mobile (16), qui peut être associé audit instrument chirurgical, et un capteur fixe (17), qui peut être fixé d'un seul tenant audit os pendant une opération chirurgicale, ledit capteur mobile (16) et ledit capteur fixe (17) étant munis de moyen de détection de position et de moyen configuré pour signaler la position détectée à une unité de traitement et de mémoire centrale,
ladite unité de traitement et de mémoire centrale comprenant des moyens d'étalonnage qui sont configurés pour détecter la position dudit capteur mobile (16) lorsqu'il est agencé dans une position fixe par rapport à une ligne passant par deux points connus dudit os et, simultanément, configurés pour détecter la position dudit capteur fixe (17) fixé d'un seul tenant audit os, et qui sont configurés pour enregistrer une telle position comme étant la position de travail initiale,
**caractérisé en ce que** ledit appareil comprend en outre un dispositif en forme de compas (15) muni de moyens de couplage (30, 31) pour se coupler de manière stable et réversible audit capteur mobile (16) et à des bras extensibles (32) dont les extrémités libres sont configurées de manière à reposer sur lesdits deux points connus dudit os pour s'agencer ainsi le capteur mobile (16) dans une relation fixe par rapport à la ligne passant par lesdits points.

2. Appareil tel que revendiqué dans la revendication 1, dans lequel ladite unité de traitement et de mémoire centrale est associée audit capteur mobile (16).

3. Appareil tel que revendiqué dans la revendication 2, comprenant en outre un dispositif en forme de pince (18) muni de moyens de couplage (33, 34) pour se coupler de manière stable et réversible audit capteur mobile (16) et de moyens de type mâchoire (35, 36) pour verrouiller en position ledit instrument chirurgical.

4. Appareil tel que revendiqué dans la revendication 3, dans lequel ladite unité de traitement et de mémoire centrale comprend des indicateurs du positionnement angulaire du capteur mobile (16) et d'un positionnement angulaire cible, lesdits indicateurs étant prévus sur une surface du capteur mobile (16) faisant face au chirurgien qui saisit l'instrument chirurgical, lorsque ledit capteur mobile (16) et ledit instrument chirurgical sont associés de manière stable à travers ladite pince (18).

5. Appareil tel que revendiqué dans la revendication 4, dans lequel lesdits indicateurs sont des indicateurs de type lumineux, les indicateurs du positionnement angulaire du capteur mobile (16) consistant en quatre LED (39) en forme de flèche agencées selon les quatre directions cardinales, dont l'activation indique la/les direction(s) dans laquelle/lesquelles le chirurgien doit incliner l'instrument pour se rapprocher du positionnement angulaire cible, et l'indicateur du positionnement angulaire cible consistant en une LED (40) qui est activée lorsque l'instrument chirurgical est correctement aligné selon le positionnement angulaire cible.

6. Appareil tel que revendiqué dans la revendication 3, dans lequel lesdits moyens de couplage (30, 31 ; 33, 34) consistent en des couplages tenon/trou avec une position de couplage univoque.

7. Appareil tel que revendiqué dans l'une quelconque des revendications précédentes, dans lequel ladite unité de traitement et de mémoire centrale comprend des moyens d'étalonnage initial qui détectent la position dudit capteur mobile (16) et dudit capteur fixe (17) lorsqu'ils sont agencés, dans une orientation préréglée, sur un même plan de référence à l'extérieur du patient et une telle position est enregistrée comme étant la position de référence initiale de ceux-ci.

8. Appareil tel que revendiqué dans la revendication 2, dans lequel ledit capteur fixe (17) est fixé d'un seul tenant sur ledit os pendant une opération chirurgicale par une broche chirurgicale.

9. Appareil tel que revendiqué dans la revendication 8, dans lequel ledit capteur fixe (17) comprend une boîte (26) dans laquelle un moyen indépendant pour la détection de sa propre position et un moyen pour signaler une telle position détectée à ladite unité de traitement et de mémoire centrale sont contenus.

10. Appareil tel que revendiqué dans la revendication 8, dans lequel ledit capteur fixe (17) comprend un support (26a) muni de multiples blocs réfléchissants (29a, 29b, 29c, 29d), et ladite unité de traitement et de mémoire centrale comprend en outre une source d'un rayonnement lumineux (42), une caméra (41) capable de capturer les images desdits blocs réfléchissants (29a, 29b, 29c, 29d) et un programme de traitement pour analyser lesdites images et fournir une détection à distance de la position exacte dudit capteur fixe (17).

11. Appareil tel que revendiqué dans l'une quelconque des revendications précédentes, dans lequel ledit os est l'os du bassin d'un être humain.

12. Appareil tel que revendiqué dans la revendication 11, dans lequel lesdits deux points connus de l'os du bassin sont les crêtes iliaques (8, 9).
